# EUROPÄISCHE PATENTANMELDUNG

(11) **EP 3 824 928 A1**
(43) Veröffentlichungstag der Anmeldung: **26.05.2021**
(21) Anmeldenummer: 20209344.9
(22) Anmeldetag: 23.11.2020
(51) Int. Cl.: A61M 5/32

(54) **SICHERHEITSKANÜLENEINHEIT, VERFAHREN ZU DEREN BEDIENUNG SOWIE DEREN VERWENDUNG**

(30) Priorität: 22.11.2019 DE 102019008125
(71) Anmelder: IME-DC GmbH International Medical Equipment - Diabetes Care, 95030 Hof (DE)
(72) Erfinder: Kamalak, Serif, 95032 Hof (DE)
(74) Vertreter: Sperschneider, Alexandra

(57) **Zusammenfassung**

Die vorliegende Erfindung betrifft Sicherheitskanüleneinheit, Verfahren zu deren Bedienung sowie deren Verwendung.

## Beschreibung

Die vorliegende Erfindung betrifft eine Sicherheitskanüleneinheit, ein Verfahren zu deren Bedienung sowie deren Verwendung.

### Stand der Technik

Aus dem Stand der Technik sind derartige Vorrichtungen bekannt, welche zur Applikation von Insulin aus einem Insulinpen verwendet werden. Bekannte Vorrichtungen erweisen sich dahingehend als nachteilig, dass sie keinen verlässlichen Sicherheitsverschluss der Kanüle bieten, nachdem die Benutzung der Vorrichtung erfolgt ist.

### Aufgabe

Daher liegt der Erfindung die Aufgabe zugrunde, eine Sicherheitsnadeleinheit bereitzustellen, welche eine verlässliche Sicherung und Entsorgung der Kanüle nach dem Injizieren des Wirkstoffs in den menschlichen oder tierischen Körper ermöglicht. Weiterhin ist es Aufgabe der vorliegenden Erfindung eine Sicherheitsnadeleinheit bereitzustellen, welche in verlässlicher Art und Weise einhändig zu bedienen ist.

### Lösung

Der Kerngedanke der vorliegenden Erfindung besteht in Sicherheitskanüleneinheit zur verletzungsfreien Entsorgung gebrauchter Injektionskanülen wenigstens aufweisend ein Kanülträgerelement, welches wenigstens eine Kanüle aufweist, eine Außenhülse zur Aufnahme des Kanülträgerelements, wobei Kanülträgerelement und Außenhülse miteinander gekoppelt sind und die Außenhülse weiterhin eine distale Öffnung aufweist, sowie wenigstens eine Innenhülse, welche gegenüber der Außenhülse axial verschiebbar und zumindest teilweise um eine Rotationsachse rotierbar ausgebildet ist, und welche zumindest nach Gebrauch der Sicherheitskanüleneinheit mittels wenigstens eines Rückstellelements abschnittsweise durch die distale Öffnung der Außenhülse herausragend angeordnet und die gebrauchte Sicherheitskanüle überspannt, wobei die Innenhülse über wenigstens einen Sicherungsmechanismus in dieser Endposition gehalten ist.

Als besonders vorteilhaft hat sich erwiesen, dass die Sicherheitskanüleneinheit wenigstens eine Innenhülse, wenigstens eine Außenhülse sowie wenigstens ein Kanülträgerelement aufweist. Diese drei Teile des Sicherheitskanüleneinheit sind wesentlich und stehen miteinander in Wirkverbindung und Funktion. Vorteilhaft sind Kanülträgerelement und Außenhülse miteinander gekoppelt. Diese Kopplung kann beispielsweise mittels Rastmechanismus, Clickmechanismus oder einer anderen Fixierung erfolgen. Die Kopplung stellt vorteilhaft sicher, dass Außenhülse und Kanülträgerelement sowohl in der unbenutzten Position als auch in der vorgesehenen Verwendung stets sicher miteinander verbunden sind und die Einheit nicht in Einzelteile zerfällt.

Somit kann sichergestellt werden, dass das Kanülträgerelement mit der daran angeordneten Kanüle stets gesichert ist.

Für die vorliegende Erfindung ist die Begrifflichkeit Kanüle nicht beschränkend zu verstehen, sodass es selbstverständlich auch denkbar ist, beispielsweise Nadeln in Analogie einzusetzen.

Weiterhin von Vorteil weist die Außenhülse eine weitere distale Öffnung auf. Unter distal ist im vorliegenden Fall vorteilhaft das Ende der Außenhülse zu verstehen, durch welche die Kanüle hindurchführbar ist.

Weiterhin weist die Sicherheitskanüleneinheit wenigstens eine Innenhülse auf, welche gegenüber der Außenhülse axial verschiebbar und zumindest teilweise um eine Rotationsachse rotierbar ausgebildet ist. Dies ist von Vorteil, da Außenhülse und Kanülträgerelement miteinander gekoppelt, vorteilhaft fest, und somit nicht in axialer Richtung gegeneinander verschiebbar ausgebildet sind. Soll nun die Kanüle nach Applikation eines Wirkstoffes, beispielsweise Insulin, geschützt werden, so übernimmt diese Aufgabe die Innenhülse. Die Innenhülse wird nach der Applikation des Wirkstoffes, in eine Sicherungsposition verbracht, in welcher die Innenhülse über die benutzte Kanüle hinausragend angeordnet ist. Somit wird die Kanüle von der Innenhülse umschlossen und überspannt. Ein mögliches Verletzungsrisiko beim Benutzer, beispielsweise beim Entsorgen der gebrauchten Sicherheitskanüleneinheit, wird signifikant reduziert bzw. gänzlich vermieden.

Hierzu ist ein Sicherungsmechanismus zwischen Innenhülse und Außenhülse vorgesehen, damit die Innenhülse in der Endposition gesichert und arretiert wird. Somit kann bei einer ungewollten Bewegung oder auch Druckbeaufschlagung auf die aus der distalen Öffnung der Außenhülse herausragende Innenhülse, diese stets in der Sicherungsposition gesichert und arretiert werden.

Die Innenhülse ist in der Außenhülse so gelagert, dass sie von einer Grundposition über eine Applikationsposition in eine Sicherungsposition verbringbar ist. In der Sicherungsposition ist die Innenhülse relativ zur Außenhülse unverschiebbar und/oder unverdrehbar gelagert. Dabei ragt die Innenhülse über die Kanüle hinaus, sodass ein unbeabsichtigtes Stechen an der Kanüle unmöglich ist.

Vorteilhaft bei der hier beschriebenen Sicherheitskanüleneinheit ist, dass die Innenhülse lediglich von der Grundposition über die Applikationsposition in die Sicherungsposition verbringbar ist, aber nicht umgekehrt. Damit wird eine sichere Entsorgung der Kanüle nach der Wirkstoffgabe sichergestellt und nachträgliche Verletzungen an der spitzen Kanüle verhindert.

Unterstützt wird der Bewegungsablauf der Innenhülse gegenüber der Außenhülse durch wenigstens ein Rückstellelement.

In der einfachsten Ausführungsform ist dieses Rückstellelement als Feder, beispielsweise Spiralfeder oder Blattfeder ausgebildet. Das Rückstellelement dient der selbsttätigen Rückstellung der Innenhülse von der Applikationsposition in die Sicherungsposition. Zum Ausbilden der Applikationsposition wird die Innenhülse mit einer Druckkraft beaufschlagt, wodurch das Rückstellelement komprimiert wird. So wird potenzielle Energie zur selbsttätigen Rückstellung des Rückstellelements gespeichert.

Weitere vorteilhafte Ausführungsformen ergeben sich aus den Unteransprüchen.

Bei einer weiteren vorteilhaften Ausführungsform ist die Innenhülse zwischen Außenhülse und Kanülträgerelement angeordnet ist. Dies ist von Vorteil, da die Innenhülse somit zwischen Außenhülse und Kanülträgerelement zwar beweglich fixiert ist, aber nicht aus der Außenhülse herausrutschen kann. Zudem wird somit eine räumlich nahe Umschließung der gebrauchten Kanüle nach deren Benutzung sichergestellt.

Bei einer weiteren vorteilhaften Ausführungsform weist das Kanülträgerelement wenigstens einen in Längsrichtung der Sicherheitskanüleneinheit gerichteten Vorsprung mit einer in Umfangsrichtung abgeschrägten Führungsfläche auf, wobei Führungsfläche und ein freies Ende der Innenhülse miteinander in Wirkverbindung stehen.

Dies ist von Vorteil, da so die Innenhülse direkt ausgelenkt werden kann. Um nun die Innenhülse auf vorbestimmbaren Bewegungspfaden zu führen, weist das Kanülträgerelement wenigstens einen in Längsrichtung der Sicherheitskanüleneinheit sich erstreckenden Vorsprung auf. Hierzu hat es sich als vorteilhaft erwiesen, wenn das Kanülträgerelement eine Basis aufweist. Vorteilhaft erfolgt die Kopplung mit der Außenhülse über diese Basis, beispielsweise über eine Ringnut oder einen Ringvorsprung, welche jeweils gegengleich untergriffen bzw. übergriffen wird.

Im einfachsten Fall sind Außenhülse und die Basis des Kanülträgerelements über einen Klickmechanismus oder Schnappmechanismus miteinander gekoppelt ausgebildet.

In Richtung distalem Ende der Außenhülse weist die Basis des Kanülträgerelements wenigstens einen, vorteilhaft mehrere Vorsprünge auf, welche sich z.B. in Längserstreckung des Kanülträgerelements und somit zugleich auch in Längserstreckung der Außenhülse und/oder quer hierzu erstrecken.

Besonders vorteilhaft sind ein, zwei oder drei Vorsprünge vorgesehen, welche in ihrer Geometrie unterschiedlich zueinander ausgebildet sein können. Es hat sich als besonders vorteilhaft erwiesen, dass die Vorsprünge der Basis wenigstens eine abgeschrägte Führungsfläche aufweisen. Diese abgeschrägte Führungsfläche ist derart ausgebildet, dass die Innenhülse, vorteilhaft mit ihrem proximalen Ende, entlang dieser abgeschrägten Führungsfläche bewegbar ausgebildet ist. Durch diese Bewegung der Innenhülse entlang der Basis wird ein Bewegungspfad beschrieben. Der Bewegung bedingt in Abhängigkeit der Form der wenigstens einen Führungsfläche eine Positionsänderung der Innenhülse. Diese kann beispielsweise in axialer Richtung verschoben werden oder aber auch um die Längsachse der Sicherheitskanüleneinheit zumindest teilweise rotiert werden.

Hierzu hat es sich in einer weiteren vorteilhaften Ausführungsform als günstig erwiesen, die Innenhülse bei Kraftbeaufschlagung entlang der abgeschrägten Endfläche bewegbar auszubilden, so dass sich während der Bewegung der Innenhülse entlang der Führungsfläche wenigstens eine Drehbewegung der Innenhülse um die Rotationsachse und/oder eine axiale Verschiebung der Innenhülse gegenüber der Außenhülse ausbildet. Durch kann in besonders einfacher Weise die Position der Innenhülse verändert werden, ohne dass die Innenhülse direkt vom Benutzer bedient werden muss.

Dies ist vorliegend nicht beschränkend zu verstehen, sodass es auch von Vorteil ist, beispielsweise einen oder mehrere Vorsprünge an der Innenhülse vorzusehen, welche dann entlang der Führungsflächen entlang bewegt werden können. Vorteilhaft erstrecken sich die Vorsprünge jeweils radial nach außen. Beispielsweise ist eine diametral einander gegenüberliegende Position denkbar. Durch das Vorsehen von zwei Vorsprüngen kann der Bewegungspfad der Innenhülse zusätzlich stabilisiert werden. Verschieben der Innenhülse kann somit leichter und verlässlicher erfolgen.

In einer weiteren vorteilhaften Ausführungsform weist das Kanülträgerelement neben der Basis weiterhin einen zumindest teilweise zylindrischer ausgebildeten Bereich auf, an dessen freiem Ende die Kanüle fest angeordnet ist. Um diesen zylindrisch ausgebildeten Bereich des Kanülträgerelements ist vorteilhaft das wenigstens eine Rückstellelement angeordnet. Um eine Fixierung des Rückstellelements einseitig sicherzustellen, ist denkbar, dass Rückstellelement an der Basis des Kanülträgerelements zu fixieren, beispielsweise zu verkleben. Dass Rückstellelement, welches beispielsweise als Feder ausgebildet sein kann, umschließt zumindest teilweise den zylindrischen ausgebildeten Bereich, vorteilhaft aber nicht die Kanüle. Somit kann sichergestellt werden, dass stets der freie Zugang zur Kanüle und deren Benutzung gewährt ist.

In einer weiteren vorteilhaften Ausführungsform weist die Außenhülse an einer inneren Mantelfläche, welche der Innenhülse zugewandt ist, wenigstens in Längsrichtung der Außenhülse verlaufende Führungsstege und/oder Vorsprünge auf, wobei zwischen zueinander benachbart angeordneten Führungsstegen Führungsnuten ausgebildet sind. Weiterhin weist die Innenhülse an einer äußeren Mantelfläche, welche der inneren Mantelfläche der Außenhülse zugewandt ist, wenigstens ein radial nach außen gerichtetes Federelement auf.

Um nun die Innenhülse während ihrer Bewegung entlang des wenigstens einen Vorsprungs der Basis des Kanülträgerelement zu stabilisieren und zu führen, weist die Außenhülse an ihrer inneren Mantelfläche wenigstens eine Profilierung auf. Die wenigstens eine Profilierung kann vorteilhaft aus mehreren, unterschiedlichen oder auch gleichen Führungsstegen und/oder Führungsnuten und/oder Vorsprüngen ausgebildet sein, mit welchen und/oder an welchen und/über welchen die Innenhülse führbar und/oder in ihrer Position veränderbar ausgebildet ist.

Besonders vorteilhaft hat es sich erwiesen, wenn die Außenhülse mehrere in ihrer Längserstreckung ausgebildete Führungsstege an der inneren Mantelfläche aufweist. Vorteilhaft ist zwischen zwei zueinander benachbart angeordneten Führungsstegen die korrespondierende Führungsnut gebildet.

In einem einfachen Ausführungsbeispiel weisen die Führungsstege einen rechteckigen Querschnitt auf. In der Folge dessen bilden die Führungsstege an der inneren Mantelfläche der Außenhülse Führungsnuten mit jeweils einem rechteckigen Querschnitt aus.

Damit nun die Innenhülse sicher und verlässlich entlang der inneren Mantelfläche der Außenhülse verschiebbar und/oder rotierbarer ist, weist die Innenhülse wenigstens ein radial nach außen hervorstehendes Federelement auf. Das wenigstens eine Federelement, welches zumindest teilweise in eine Führungsnut der Außenhülse eingreift, weist in einem einfachen Ausführungsbeispiel ebenfalls einen rechteckigen Querschnitt auf und kann so verlässlich in der Führungsnut axial verschoben werden. Das Federelement entwickelt vorteilhaft eine Federkraftwirkung radial nach außen, sodass das Federelement wenigstens zum Teil in und gegen die entsprechende Führungsnut gedrückt wird. Dadurch wird die Innenhülse, von der aus sich das Federelement erstreckt, sicher entlang der entsprechenden Führungsnut geführt.

Einer weiteren vorteilhaften Ausführungsform weist die Innenhülse wenigstens zwei einander diametral gegenüberliegende Federelemente auf, welche bei axialer Verschiebung der Innenhülse in den Führungsnuten der Außenhülse und bei Rotationsbewegung der Innenhülse über Führungsstege oder Führungsvorsprünge der Außenhülse hinweg rotierbar sind.

Besonders vorteilhaft hat sich erwiesen, wenn zwei einander diametral gegenüberliegende Federelemente an der Innenhülse vorgesehen sind. Auch hierdurch kann eine verbesserte Führung erzielt werden.

Besonders vorteilhaft ist das wenigstens eine Federelement in seiner Erstreckung komplementär zu den Führungsvorsprüngen und/oder den Führungsnuten und/oder den Vorsprüngen der inneren Mantelfläche der Außenhülse ausgebildet. Dies bedeutet, dass die Innenhülse über das wenigstens eine Federelement auf den Führungsvorsprüngen entlang axial verschoben werden kann. Hierzu wird eine Kraft wie auf das Federelement radial nach innen ausgeübt, sodass das wenigstens eine Federelement nach innen geführt ist und die verbleibende Wandung der Innenhülse den Führungsvorsprung zumindest teilweise seitlich umgreift.

In einem anderen möglichen Bewegungsablauf ist denkbar, dass das Federelement unausgelenkt verbleibt und durch die radial nach außen hervorstehenden Position in einer Führungsnut axial verschoben werden kann. Das Federelement ist in diesem Bewegungsablauf zwischen zwei Führungsvorsprüngen angeordnet und entlang diesen in der Position axial verschiebbar.

Schließlich kann auch eine Rotationsbewegung der Innenhülse notwendig und bedingt sein. Dies wird dadurch ermöglicht, dass das wenigstens eine Federelement in seiner ursprünglich nach außen hervorstehenden Position mit einer Druckkraft beaufschlagt wird, da die Rotationsbewegung vorgibt, dass Federelement und somit Innenhülse von einer ersten Führungsnut in eine zweite weitere Führungsnut zu überführen. Hierzu ist es notwendig, dass wenigstens ein Führungsvorsprung überwunden werden muss. Hierzu erweist sich das wenigstens eine Federelement als besonders vorteilhaft, da dieses durch die Druckkraftbeaufschlagung nach innen verlagert wird, wodurch die Rotation über den wenigstens einen Führungsvorsprung ermöglicht und erleichtert wird. Ist der Führungsvorsprung dann überwunden, wird aufgrund der Rückstellkraft des Federelements dieses wieder radial nach außen in die ursprüngliche Position geführt, sodass dieses in die zweite, weitere Führungsnut eingreift und sicher in dieser gehalten wird.

Einer weiteren vorteilhaften Ausführungsform ist der Sicherungsmechanismus nach Gebrauch der Kanüle durch eine formschlüssige und/oder kraftschlüssige Verbindung zwischen wenigstens einem Federelement und wenigstens einem Arretierungsvorsprung ausgebildet ist, wobei der Arretierungsvorsprung an der inneren Mantelfläche der Außenhülse angeordnet ist.

Wurde die Sicherheitskanüleneinheit nunmehr benutzt, beispielsweise um einen Wirkstoff an einen Patienten zu verabreichen, ist es relevant, dass die gebrauchte Kanüle geschützt ist. Dies verhindert, dass sich der Benutzer im Nachgang sticht oder auch andere Personen vor Verletzungen geschützt sind.

Hierzu hat es sich als besonders vorteilhaft erwiesen, wenn das wenigstens eine Federelement der Innenhülse mit einem Arretierungsvorsprung, welcher weiterhin ebenfalls an der inneren Mantelfläche der Außenhülse angeordnet ist, in Wechselwirkung tritt. Besonders vorteilhaft ist dieser Arretierungvorsprung radial nach innen erstreckend ausgebildet. Wird nun die Innenhülse, genauer gesagt das wenigstens eine Federelement durch die oben beschriebene axiale Verschiebung in Längsrichtung der Sicherheitskanüleneinheit durch die distale Öffnung der Außenhülse geführt, sodass die gebrauchte Kanüle vollständig von der Innenhülse umschlossen ist, muss genau diese Position der Innenhülse arretiert und gesichert werden. Hierzu weist die innere Mantelfläche der Außenhülse wenigstens einen Arretierungvorsprung pro Federelement auf. Während der axialen Verschiebung der Innenhülse trifft das wenigstens eine Federelement auf den jeweiligen Arretierungsvorsprung, sodass durch die einwirkende Kraft auf das Federelement dieses radial nach innen geführt wird. Somit kann das Federelement und die gesamte Innenhülse den Arretierungsvorsprung überwinden. Mit Beendigung der Kraft springt das wenigstens eine Federelement wieder in seinem Ursprungsposition zurück und wird sicher von dem korrespondierenden Arretierungsvorsprung untergriffen. Die Endposition ist gesichert, in welcher die innere Hülse die gebrauchte Kanüle vollständig umschließt. Verletzungen sind somit nicht mehr möglich.

Der erste Arretierungsvorsprung weist vorteilhaft eine Keilform aus, sodass dieser als Rampe für das Federelement ausgebildet ist. Wird die Innenhülse entlang der Vorsprünge des Kanülträgerelements bewegt, erfährt die Innenhülse beispielsweise eine Kraftbeaufschlagung zur Rotation. Durch die eingeleitete Rotationsbewegung erfährt auch das wenigstens eine Federelement eine Kraftbeaufschlagung durch den benachbart hierzu angeordneten Führungssteg, welcher zunächst als Begrenzung wirkt. Dabei ist der erste Arretierungsvorsprung entlang des Führungsstegs als Unterbrechung desselben ausgebildet. Besonders vorteilhaft hat sich die Keilform erwiesen, so dass die Rampe in Rotationsrichtung zunehmend ausgebildet ist. Hierdurch kann das wenigstens eine Federelement auf den Keil geführt werden und diesen überwinden. Das wenigstens eine Federelement kann somit von einer ersten Führungsnut in eine zweite Führungsnut verbracht werden. Durch die Keilform ist diese Rotationsbewegung irreversibel. Eine Rückrotation wird durch das hohe Keilende verhindert. Die Rampe dient der erleichterten Führung mit weniger Kraftaufwand während der Rotation und der zugleich sicheren Halterung, um eine Rückrotation zu verhindern.

Weiterhin ist in der Mantelfläche der Außenhülse zwischen zwei Führungsstegen wenigstens ein weiterer, zweiter Arretierungsvorsprung angeordnet. Vorteilhaft ist dieser orthogonal zu den Führungsstegen angeordnet, sodass das wenigstens eine Federelement der Innenhülse durch eine Längsverschiebung, welche beispielsweise durch die Rückstellkraft des Rückstellelements ausgeführt wird, über den zweiten Arretierungsvorsprung hinwegführbar ist. Dieser untergreift anschließend wenigstens das Federelement und/oder auch weitere Teile der Innenhülse.

Auch hier kann der zweite Arretierungsvorsprung eine Keilform aufweisen, sodass dieser für das Federelement eine Rampe ausbildet. Bedingt beispielsweise die Rückstellkraft des Rückstellelements, dass die Innenhülse in Richtung distalem Ende bewegt werden soll, so wird das Federelement über den zweiten keilförmigen Arretierungsvorsprung geführt. Nach Überwindung des zweiten Arretierungsvorsprung gleitet das Federelement weiter in der entsprechenden Führungsnut, kann allerdings nicht mehr zurückgeführt werden, da der zweite Arretierungsvorsprung beim Zurückführen das Federelement untergreift. Die entsprechende Führungsnut bildet gemeinsam mit dem zweiten Arretierungsvorsprung eine Einbahnstraße für das Federelement aus.

Bei einer weiteren vorteilhaften Ausführungsform ist das Rückstellelement in einer Ausgangsposition bereits vorgespannt ausgebildet ist. Unter Ausgangsposition ist vorliegend die Position zu verstehen, in welcher die Sicherheitskanüleneinheit unbenutzt vorliegt. Durch die bereits vorgesehene Vorspannung des Rückstellelement kann der spätere Kraftaufwand zum Spannen des Rückstellelements verringert werden. Dies ist insbesondere für ältere Menschen mit weniger Kraft von Vorteil.

In einer weiteren, vorteilhaften Ausgestaltung der Einheit ist das Rückstellelement eine Druckfeder, die konzentrisch zum zylindrischen Bereich des Trägerelements in Längsrichtung der Einheit angeordnet ist. Im einfachsten Fall ist vorteilhaft die Druckfeder auf den zylindrischen Bereich aufgeschoben. Mit dem einen Ende stößt die Druckfeder dabei an dem Kanülträgerelement selbst an und mit dem gegenüberliegenden Ende an der Innenhülse bzw. an einem radial nach innen abstehenden Absatz an der inneren Mantelfläche der Innenhülse.

So kann mit Druckbeaufschlagung auf die Innenhülse das Rückstellelement in Form der Druckfeder gespannt werden.

Weiterhin betrifft die vorliegen Erfindung eine Einhandverfahren zur Bedienung eines Wirkstoffverabreichungsmittel, welches wenigstens eine Sicherheitskanüleneinheit aufweist.

Bei dem Einhandverfahren zur Betätigung eines Wirkstoffverabreichungsmittels mit wenigstens einer Sicherheitskanüleneinheit wird die Sicherheitskanüleneinheit distal an dem Wirkstoffverabreichungsmittel angeordnet ist, beispielsweise angeklickt. Die Sicherheitskanüleneinheit erfährt eine Druckkraftbeaufschlagung, so dass beispielsweise die Kanüle aus der Außenhülse herausgeführt wird. Zugleich kann auch die Innenhülse axial verschoben werden und durch die distale Öffnung der Außenhülse herausgeführt werden. Vor Applikation des Wirkstoffes ist die Kanüle stets der Innenhülse überstehend ausgebildet.

Soll eine hier beschriebene Sicherheitskanüleneinheit zum sicheren Verschließen einer gebrauchten Kanüle benutzt werden, so wird die Auslenkung der Innenhülse aus deren Grundposition, auch Ausgangsposition, zunächst bedingt. Erfährt danach die Innenhülse eine Druckkraftbeaufschlagung, beispielsweise durch das Aufsetzen oder Aufdrücken auf die Hautoberfläche, so wird die Innenhülse aus ihrer Grundposition ausgelenkt und nach unten in Richtung Boden der Außenhülse geführt. Hierdurch wird das Rückstellelement komprimiert. Vorteilhaft wird die Innenhülse nicht nur in linearer Position verschoben, sondern zugleich auch um einen vorbestimmbaren Winkel von beispielsweise 1° bis 45°, vorteilhafter von 2°, 3°, 4°, $°, 5°, 6°, 7°, 8°, 9°, 10°, 11°, 12°, 13°, 14°, 15°, 16°, 17°, 18°, 19°, 20°, 21°, 22°, 23°, 24°, 25°, 26°, 27°, 28°, 29°, 30°, 31°, 32°, 33°, 34°, 35°, 36°, 37°, 38°, 39°, 40°, 41°, 42°, 43°, 44° oder 45° rotiert. Diese Rotationswinkel sind vorteilhaft, da hiermit durch geringe Rotationsbewegungen effektive Positionsänderungen erzeugen lassen. Vorteilhaft erfolgt die Rotation um die Längsachse der Einheit. Sowohl axiale Verschiebung als auch Rotation werden vorteilhaft durch die Bewegung der Innenhülse entlang der Vorsprünge des Kanülträgerelements ausgebildet.

Mit Rotation der Innenhülse wird das wenigstens eine Federelement der Innenhülse aus seiner Grundposition ausgelenkt und mit einer Druckkraft beaufschlagt. Mit dieser Druckkraftbeaufschlagung in Kombination mit der Rotation kann die Innenhülse um den oben beschriebenen Winkel rotiert werden und über wenigstens einen Führungssteg der Außenhülse hinweg rotieren. Vorteilhaft ist der Führungssteg in seiner Geometrie von dem rampenartigen ersten Arretierungsvorsprung unterbrochen, Mit Beendigung der Druckkraftbeaufschlagung auf die Innenhülse wird das Rückstellelement entlastet und kann somit in seine ursprüngliche Form zurückkehren. Dies bedeutet zugleich, dass durch die Rückstellkraft auch die Innenhülse in Richtung Kanülenaustrittsfläche rückgeführt wird. Vorteilhaft erfolgt diese Rückführung linear. Hierzu weist die Außenhülse vorteilhaft zwei sich in Längserstreckung der Außenhülse erstreckende Stege auf, welche zugleich der Führung der Innenhülse dienen. Um nun eine besonders sichere Endposition der Kanüle zu gewährleisten, sodass nach deren Benutzung keine Verletzungsgefahr möglich ist, sind die beiden Führungsstege, zwischen welchen das wenigstens eine Federelement der Innenhülse zur Sicherungsposition geführt wird, mit einem Quersteg als weiteren Arretierungsvorsprung verbunden. Vorteilhaft ist der Quersteg in der oberen Hälfte der Außenhülse vorgesehen.

Besonders vorteilhaft kann durch die Rückstellkraft des Rückstellelements das Federelement der Innenhülse erneut in seiner Position ausgelenkt werden und vorteilhaft über den Quersteg geführt werden. Mit Beendigung dieser Kraftbeaufschlagung wird das Federelement der Innenhülse wieder in seine ursprüngliche Position zurückkehren wollen, sodass der Quersteg das Federelement der Innenhülse untergreift und hierdurch sichert. Die Innenhülse wird dadurch verlässlich und dauerhaft in ihrer Position gehalten, sodass keine Verletzungen mit der benutzten Kanüle möglich werden. Folglich kann der hier beschriebene Mechanismus auch vorteilhaft als Sicherungsmechanismus beschrieben werden. Quersteg ist hierbei synonym zum Arretierungsvorsprung zu verstehen.

Weiterhin ist denkbar, dass die Außenhülse, an der der Kanüle gegenüberliegenden Seite mit einem Sicherheitsverschluss verschließbar ausgebildet ist. Dieser Sicherheitsverschluss kann beispielsweise auch für die Sterilisierung vorgesehen sein.

In der Grundposition ragt die Kanüle über die Innenhülse hinaus. So kann die Kanüle leicht an eine entsprechende Stelle für die Medikamentenapplikation herangeführt werden. Es ist allerdings auch denkbar, dass die Innenhülse der Kanüle übersteht, sodass ein unbeabsichtigtes Stechen mit der Kanüle verhindert wird.

In der Applikationsposition ist die Innenhülse soweit in Richtung Basis des Kanülträgerelement verschoben, dass die Kanüle freiliegt.

Damit die Innenhülse die Sicherungsposition einnehmen kann, wird diese durch das Rückstellelement wieder von dem Kanülträgerelement weggeführt, wobei die Innenhülse aufgrund des Federelements, welches in den Führungsnuten geführt wird, sowie der Arretierungsvorsprünge, der wenigstens einen Führungsfläche an dem Kanülträgerelement sowie optional der an der innen Mantelfläche der Außenhülse hervorstehenden Führungsnase in einer vorbestimmbaren Bewegungsbahn bewegt wird. Dabei wird die Innenhülse selbsttätig in die Sicherungsposition verbracht. Dabei überragt die Innenhülse die Kanüle und ist auch nicht mehr verschieb- und/oder verdrehbar. Dadurch wird vorteilhafterweise ein versehentliches Stechen an der Kanüle effektiv verhindert.

Besonders vorteilhaft an diesem Verfahren ist, dass die Einheit mit einer Hand bedient wird. Eine einhändige Bedienung ist besonders intuitiv und erleichtert die Medikamentenapplikation erheblich. Außerdem ist eine einfache einhändige Bedienung besonders vorteilhaft für motorisch eingeschränkte Menschen

Die Bewegung der Innenhülse innerhalb der Außenhülse von der Grundposition über die Applikationsposition in die Sicherungsposition ist durch die Arretierungsvorsprünge unumkehrbar, da bei entgegengesetzter Bewegungsrichtung die Arretierungsvorsprünge das Federelement der Innenhülse untergreifen und so in seiner entsprechenden Bewegungsrichtung blockieren. Dadurch kann ein unbeabsichtigtes Zurückschieben der Innenhülse über die Kanüle nach deren Verwendung vorteilhaft unterbunden werden.

Weiterhin betrifft die Erfindung ein Wirkstoffverabreichungsmittel mit einer Sicherheitskanüleneinheit.

Den betrifft die Erfindung die Verwendung der Sicherheitskanüleneinheit zur Bestückung von Insulinspritzen, Insulinpens oder dergleichen.

Besonders vorteilhaft sind Außenhülse und Innenhülse aus Kunststoff ausgebildet. Dies ist von Vorteil, da sich einfach und schnell die komplette Einheit sterilisieren und verpacken lässt. Zudem ist auch das durch den Kunststoff bedingt geringe Gewicht der Einheit von Vorteil. Die ist selbstverständlich nicht begrenzend zu verstehen, sodass es auch denkbar ist, dass neben Kunststoff auch weitere Materialien zum Einsatz kommen.

Zu Benutzung der hier beschriebenen Sicherheitskanüleneinheit mit einem Insulinpen wird die Außenhülse auf den entsprechenden Pen aufgesteckt. Nach Entfernung einer optionalen Verschlusskappe auf Seite der Sicherheitskanüleneinheit kann dann diese auf der Haut platziert werden und über eine Druckkraftbeaufschlagung auf den Pen die Injektion ausgelöst werden.

Weiterhin betrifft die vorliegende Erfindung auch eine Verpackungseinheit zur sterilen und/oder sicheren Verwahrung der Sicherheitskanüleneinheit, wobei die Verpackungseinheit wenigstens eine Abdeckkappe, die auf der Sicherheitskanüleneinheit angeordnet ist und diese steril verschließt, aufweist. Die Abdeckkappe ist im Wesentlichen zylinderförmig ausgebildet, wobei eine Seite verschlossen und ihr gegenüberliegend eine Öffnung angeordnet ist. Über diese Öffnung kann eine Sicherheitskanüleneinheit aufgenommen werden. Weiterhin kann die Abdeckkappe auch als Sicherheitsverschluss aufgefasst werden. Abdeckkappe und Sicherheitsverschluss sind hierbei synonyme Begriffe.

Besonders vorteilhaft ist die hier beschriebene Einheit auch als Sicherheitspenkanüle zu bezeichnen.

Es ist jedoch auch denkbar, dass neben Insulin auch andere Medikamente über die genannte Vorrichtung appliziert werden können. Vorteilhaft ist hierbei, dass die Einheit nach der Medikamentenapplikation selbsttätig in die Sicherungsposition verbracht wird, sodass von der benutzten Kanüle keine Verletzungsgefahr mehr ausgeht, da die Innenhülse die Kanüle bzw. die Kanülenspitze schützend umgibt. Weiterhin ist besonders vorteilhaft, dass die Verwendung mit nur einer Hand erfolgt, was besonders vorteilhaft ist, da einerseits motorisch eingeschränkte Personen die genannte Vorrichtung verwenden können und andererseits die Verwendung einfach und intuitiv ist. Durch die festgelegte Abfolge von Grundposition, Applikationsposition und Sicherungsposition der Innenhülse während der Medikamentenapplikation, die unumkehrbar ist, wird das Risiko einer falschen Benutzung der Einheit weitestgehend verhindert.

Weitere Vorteile, Merkmale und Ausgestaltungsmöglichkeiten ergeben sich aus der nachfolgenden Figurenbeschreibung von nicht einschränkend zu verstehenden Ausführungsbeispielen.

### Kurzbeschreibung der Zeichnungen

Hierbei zeigt
- FIG 1: eine perspektivische Ansicht einer Verpackung für eine Sicherheitskanüleneinheit;
- FIG 2: eine perspektivische Ansicht einer Sicherheitskanüleneinheit;
- FIG 3: eine perspektivische Ansicht einer Sicherheitskanüleneinheit in Sicherungsposition;
- FIG 4: eine perspektivische Ansicht einer Außenhülse;
- FIG 5: perspektivische Ansichten von Innenhülsen;
- FIG 6: eine perspektivische Ansicht eines Rückstellelements;
- FIG 7: eine perspektivische Ansicht eines Kanülträgerelements;
- FIG 8: zwei Schnittansichten von Außenhülsen;
- FIG 9a: bis e weitere Schnittansichten von Teilen der Sicherheitskanüleneinheit in unterschiedlichen Positionen;
- FIG 10a: eine Schnittansicht einer alternativen Ausführungsform der Innen- und Außenhülse in der Grundposition,
- FIG 10b: eine Schnittansicht einer alternativen Ausführungsform der Innen- und Außenhülse mit daran angeordnetem Trägerelement mit Kanüle in der Grundposition,
- FIG 11a: eine Schnittansicht einer alternativen Ausführungsform der Innen- und Außenhülse kurz vor der Applikationsposition,
- FIG 11b: eine Schnittansicht einer alternativen Ausführungsform der Innen- und Außenhülse mit daran angeordnetem Trägerelement mit Kanüle kurz vor der Applikationsposition,
- FIG 12a: eine Schnittansicht einer alternativen Ausführungsform der Innen- und Außenhülse in der Sicherungsposition,
- FIG 12b: eine Schnittansicht einer alternativen Ausführungsform der Innen- und Außenhülse mit daran angeordnetem Trägerelement mit Kanüle in der Sicherungsposition,

In den Zeichnungen mit gleichen Bezugszeichen versehene Elemente entsprechen im Wesentlichen einander, sofern nichts anderes angegeben ist. Darüber hinaus wird darauf verzichtet, Bestandteile zu zeigen und zu beschreiben, welche nicht wesentlich zum Verständnis der hierin offenbarten technischen Lehre sind. Im Weiteren werden nicht für alle bereits eingeführten und dargestellten Elemente die Bezugszeichen wiederholt, sofern die Elemente selbst und deren Funktion bereits beschrieben wurden oder für einen Fachmann bekannt sind. Ferner wird darauf hingewiesen, dass die gezeigten Komponenten nicht maßstabsgerecht abgebildet sind. Die Größenverhältnisse der einzelnen Komponenten zueinander sind ebenfalls nicht maßstabsgerecht abgebildet.

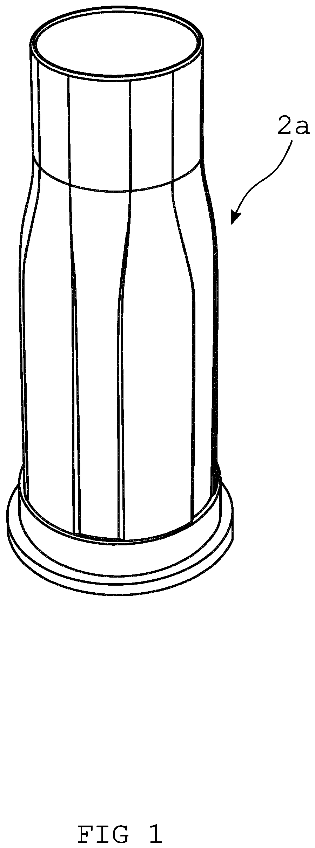

In der FIG 1 ist eine perspektivische Ansicht einer Verpackung 2a für die Sicherheitskanüleneinheit gezeigt. In der Abbildung erstreckt sich die zylindrische Form der Verpackung 2a von einer Basis, die als ein verstärkter Ring ausgebildet ist, nach oben hin verjüngend.

Weiterhin weist die Verpackung 2a in der gezeigten Ausführungsform zwei Bereiche mit unterschiedlichem Außenradius der zylindrischen Form auf, wobei der in der Abbildung obere Bereich einen kleineren Radius aufweist als der untere Bereich. Zwischen den unterschiedlichen Bereichen verläuft der Übergang vom größeren zum kleineren Radius konisch und bildet somit einen weichen Übergang zwischen den unterschiedlichen Außenradien aus. Weiterhin ist die äußere Mantelfläche der Verpackung 2a in Längsrichtung der zylindrischen Form zum Teil leicht abgeflacht.

In der Grundposition schützt die Verpackung 2a die Sicherheitskanüleneinheit 1 und insbesondere die Kanüle 12 vor Verschmutzungen und hält die Einheit steril. In der Schutzposition bietet die Verpackung 2a einen weiteren, redundanten Schutz gegenüber der benutzten Kanüle 12.

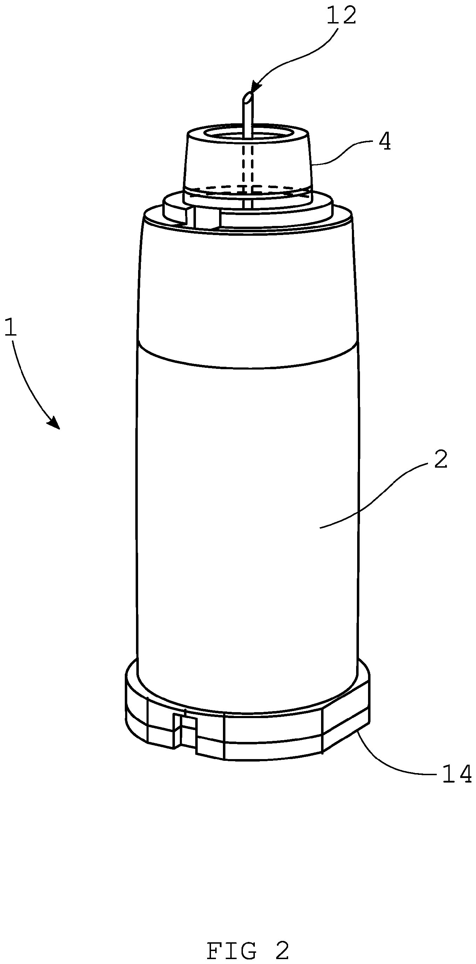

FIG 2 zeigt eine perspektivische Ansicht einer Sicherheitskanüleneinheit 1. Diese Einheit 1 weist eine Außenhülse 2, eine aus der Außenhülse 2 hervorstehende Innenhülse 4 sowie ein Kanülträgerelement 14 auf. Von Letzterem ist lediglich die Basis zu sehen, welche mit der Außenhülse verbunden ist und eine gemeinsame radiale Kontaktfläche ausbildet.

Aus der Innenhülse 4 hervorstehend ist die Kanüle 12 gezeigt.

Die hier gezeigt Ausführung kann als Ausgangsposition verstanden werden, also, bevor die Applikation eines Wirkstoffs über die Kanüle stattfindet.

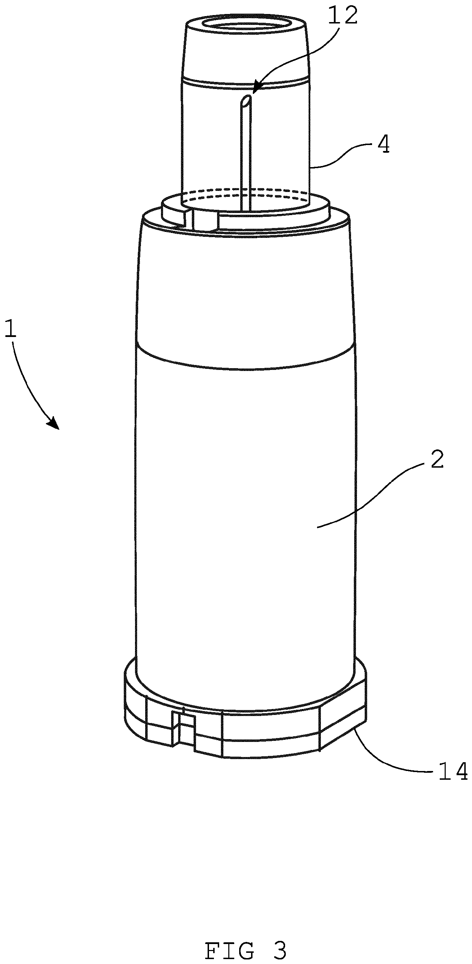

FIG 3 zeigt die Sicherheitskanüleneinheit 1 in der Sicherungsposition. Die Abbildung entspricht der FIG 2 mit dem Unterschied, dass die Innenhülse 4 weiter aus der Außenhülse 2 nach oben hin herausragt, sodass die Kanüle 12 sicher von der Innenhülse 4 überspannt ist. Damit ist die Kanüle 12 insgesamt sicher in der Sicherheitskanüleneinheit 1 aufgenommen und es geht keine Verletzungsgefahr von ihr aus.

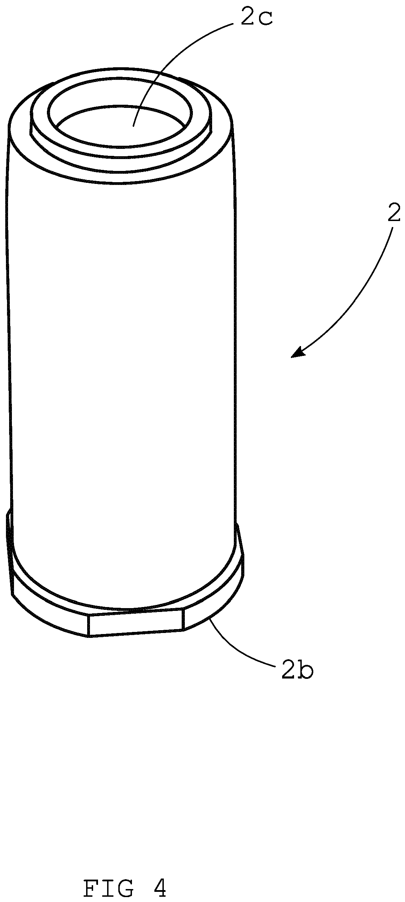

Fig. 4 zeigt eine perspektivische Ansicht einer Außenhülse 2. Die Außenhülse 2 weist ebenfalls eine zylindrische Form auf. Am unteren Bereich ist eine Verdickung 2b ausgebildet. Am oberen distalen Ende weist die Außenhülse 2 eine Öffnung 2c auf, durch die eine Innenhülse (nicht gezeigt) wenigstens zum Teil durchgeführt werden kann.

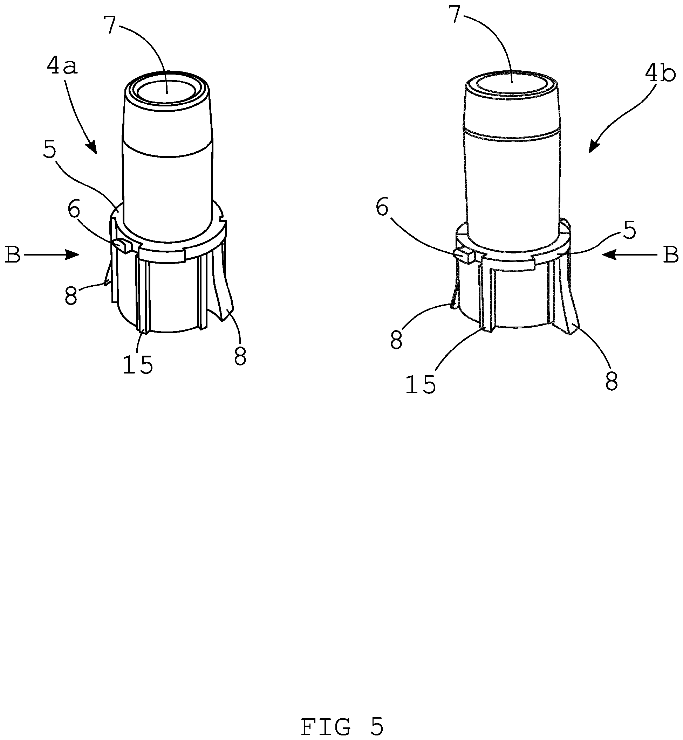

Fig. 5 zeigt zwei mögliche Ausführungen einer Innenhülse 4.

Die Innenhülse 4 weist zumindest teilweise eine zylindrische Form auf. Insgesamt weist die Innenhülse 4 drei Bereiche auf. Der in der Abbildung unterste Bereich B ist profiliert ausgebildet. An der Mantelfläche sind hierbei zwei sich diametral gegenüberliegende Federelemente 8 angeordnet. Sie erstrecken sich von einem Vorsprung 5, welcher den oberen Abschluss des unteren Bereichs B ausbildet, in Längsrichtung nach unten und erstrecken sich in ihrem Verlauf radial nach außen. An ihrem unteren Ende weisen die Federelemente 8 jeweils eine Verdickung auf. Die Federelemente 8 sind flexibel ausgebildet und können ein einwirkender Kraftbeaufschlagung, von der hier gezeigten Grundposition radial nach innen in den unteren Bereich hinein verschwenkt werden. Wird die Kraftbeaufschlagung beendet, führt die zungenartige Ausbildung der Federelemente 8 dazu, dass diese über die Rückstellkraft wieder in die hier gezeigte Grundposition zurückgeführt werden. Die Federelemente 8 federn wieder in die Grundposition zurück.

Weiterhin weist die Innenhülse 4 wenigstens ein Vorsprung 15 auf, welcher die Form eines Stegs hat. Dieser erstreckt sind vom ringförmigen Vorsprung 5 in Längsrichtung der Innenhülse 4 nach unten und schließt mit dem unteren Ende der Innenhülse 4 ab.

Am Umfang des ringförmigen Vorsprungs 5 ist weiterhin wenigstens ein weiterer Vorsprung 6 angeordnet, welcher sich radial nach außen hin erstreckt. Der Vorsprung ist für die Führung der Innenhülse 4 innerhalb der Außenhülse (hier nicht gezeigt) von Vorteil.

Am oberen, distalen Ende kann die Innenhülse 4 eine Öffnung 7 aufweisen, durch die eine Kanüle 12 (nicht gezeigt) geführt werden kann. Die zylindrische Form läuft im oberen Bereich der Innenhülse 4 leicht konisch zur Öffnung hin zu.

Weiterhin ist die Innenhülse 4 in ihrer Größe und Geometrie derart ausgebildet, dass sie wenigstens zum Teil in die Außenhülse 2 aus FIG 4 aufgenommen werden kann. In dieser Außenhülse 2 ist die Innenhülse 4 drehbar und axial verschiebbar gelagert angeordnet. Auf der linken Seite der Fig. 5 ist eine erste Ausführungsform einer Innenhülse 4a gezeigt. Der untere Bereich B ist länger ausgebildet als bei der zweiten Ausführungsvariante 4b. Zudem ist bei der zweiten Variante 4b der sich vom unteren Bereich B nach oben erstreckende Korpus zylindrisch oder sich nach oben konisch aufweitend ausgebildet. Beide Varianten 4a, 4b weisen die Öffnung 7 für die Kanüle auf.

Weiterhin ist in Variante 4b das Federelement 8 in radialer Erstreckung verlängert ausgebildet und überragt das vom Betrachter aus untere Ende der Innenhülse 4. Hierdurch wird zusätzliche Rückstellkraft gewonnen und zugleich eine verbesserte Führung der Innenhülse 4 an der Außenhülse 2 entlang gewährleistet.

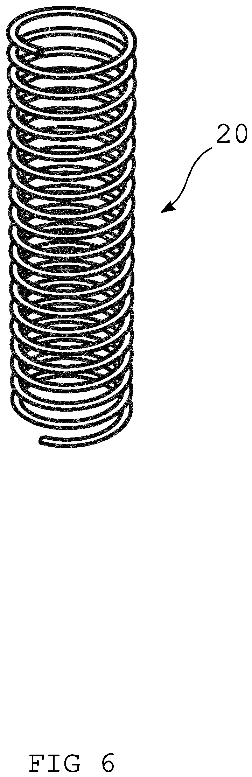

FIG 6 zeigt ein Rückstellelement 20 in perspektivischer Ansicht. Das Rückstellelement 20 ist im einfachsten Fall ist als eine Spiralfeder ausgebildet. In der gezeigten Ausführung ist das Rückstellelement 20 eine Druckfeder.

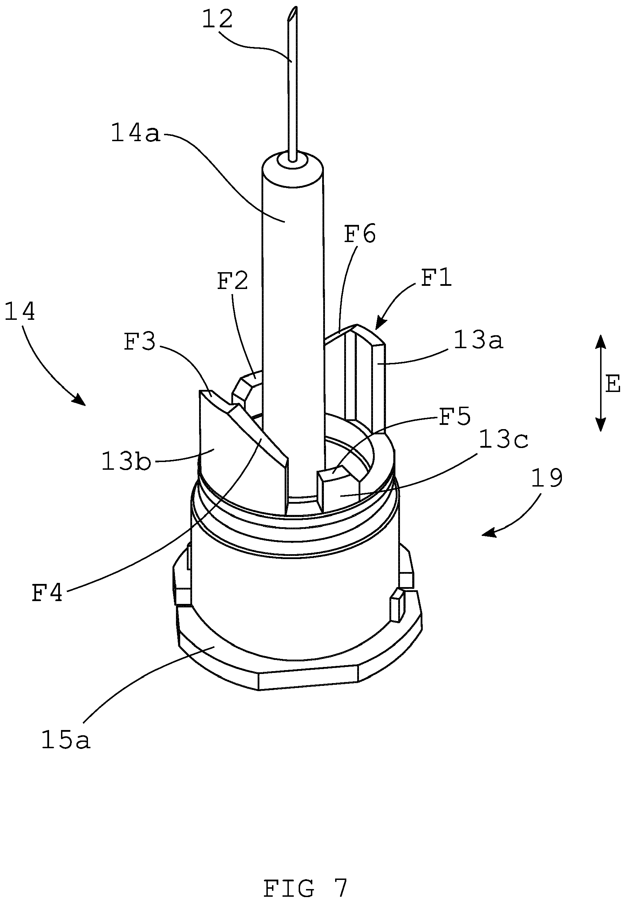

FIG 7 zeigt ein Kanülträgerelement 14 mit Kanüle 12 in der Ansicht von schräg oben. Das Kanülträgerelement 14 weist eine Basis 19 auf, welche eine Materialverdickung 15a als unteren Abschluss aufweist. Diese Materialverdickung 15a bildet im zusammengeführten Zustand von Kanülträgerelement 14 und Außenhülse 2 (wie in Fig. 2 gezeigt) mit der Außenhülse 2 eine gemeinsame Kontaktfläche aus. Die Basis 19 kann auch als Sockel bezeichnet werden.

In Längserstreckung E schließen sich an die Basis 19 mehrere Vorsprünge 13a-13c an, welche jeweils für sich eine oder auch mehrere Führungsflächen F1 bis F6 ausbilden. Entlang dieser Führungsflächen F kann die Innenhülse 4 entlang bewegt werden. Die unterschiedliche Neigung der Führungsflächen sowie die unterschiedliche Höhe der Vorsprünge 13 bedingt die Positionsänderung der Innenhülse 4 in axialer Richtung, beispielsweise in Längsrichtung E und/oder in Rotation um die sich in Längsrichtung E erstreckende Rotationsachse R.

Um den zylindrischen Bereich 14a, an welchem endseitig die Kanüle 12 angeordnet ist, kann das Rückstellelement 20 aus FIG 5 konzentrisch angeordnet werden.

Für die Montage der Sicherheitskanüleneinheit 1 kann die Innenhülse 4 aus FIG 5 ebenfalls auf und/oder an den zylindrischen Bereich 14a angeordnet werden. Danach wird auf das Kanülträgerelement 14 die Außenhülse 2 aus FIG 4 aufgeschoben, sodass gleichzeitig die Innenhülse 4 in der Außenhülse 2 wenigstens teilweise aufgenommen ist. Vorteilhaft verrasten oder verklicken Außenhülse 2 und Kanülträgerelement 14 miteinander und sind hierdurch fixiert. Gleiches gilt für die Innenhülse 4, welche zwar beweglich innerhalb der Außenhülse 2 ist, aber durch deren reduzierte Öffnung 2c nicht aus dieser herausgleiten kann.

Zum Schutz der Komponenten der Sicherheitskanüleneinheit kann ebenfalls die Verpackung aus FIG 1 über die gesamte Sicherheitskanüleneinheit geschoben werden, sodass diese vor Beschädigung o.Ä. geschützt ist.

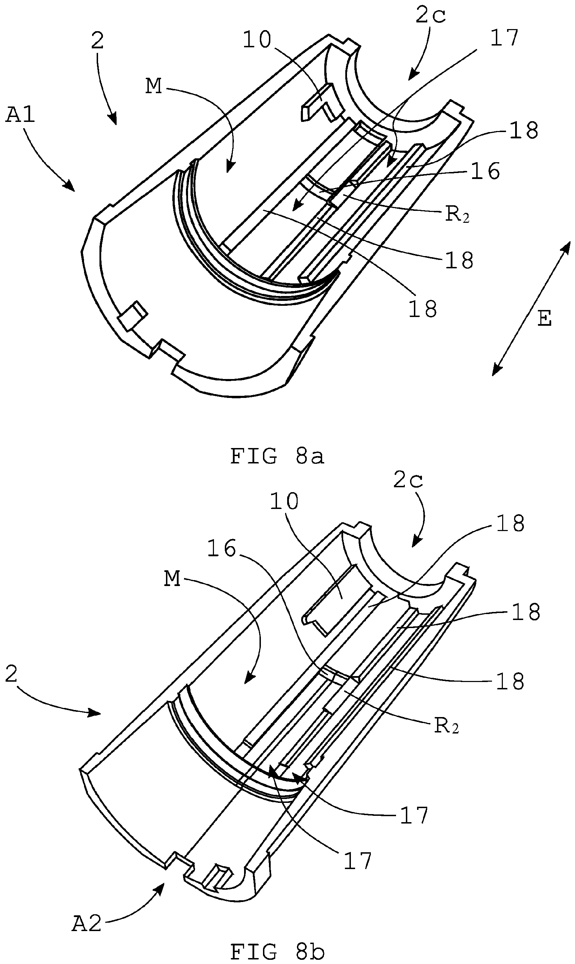

Die FIG 8a, 8b zeigt eine Schnittansicht einer Außenhülse 2 in zwei unterschiedlichen Ausführungsvarianten.

Auf der Innenseite, oder auch Mantelfläche M, der Außenhülse 2 sind Führungsstege 18 angeordnet, die in ihrem Querschnitt jeweils eine Rechteckform aufweisen und in Längsrichtung E verlaufen. Die Führungsstege 18 sind dabei parallel zueinander angeordnet und bilden entsprechende Führungsnuten 17 aus.

Weiterhin ist vorteilhaft ein Führungssteg 18 rampenförmig unterbrochen ausgebildet. Diese Rampe R₂ dient dazu, die Rotationsbewegung des Federelements (nicht gezeigt) zu erleichtern und einen Wechsel zwischen zwei Führungsnuten zu ermöglichen.

Zwischen zwei Führungsnuten 17 ist ein erster Arretierungsvorsprung R angeordnet, welcher den jeweiligen Führungssteg 18 in Längsrichtung unterbricht. Der Arretierungsvorsprung R kann keilförmig ausgebildet sein, sodass dieser von einer Seite aus bspw. ein in die Führungsnut 17 formschlüssig eingepasstes Federelement 8 untergreift und es so an einer Rotation hindert. Eine reversible Führung des Federelements ist dann nicht mehr möglich.

Weiterhin ist eine Führungsnase 10 vorgesehen. Hierdurch kann die Positionsänderung der Innenhülse nochmals kontrolliert und stabilisiert werden. Die Bewegung der Innenhülse wird durch den Umfang der Führungsnase 10 mit ausgebildet. Die Führungsnase 10 in FIG 8a ist L-Förmig ausgebildet, wobei ein Schenkel in Umfangsrichtung der Außenhülse 2 und der andere Schenkel in Längserstreckung E der Außenhülse 2 verläuft. Bei Ausführungsvariante A1 läuft ein Schenkel der Führungsnase 10 zu seinem äußeren Ende hin spitz zu. Bei Ausführungsvariante A2 ist die Führungsnase 10 eher blockartig ausgebildet. Vorteilhaft passen Ausführungsvariante A1 der Außenhülse 2 und die Innenhülse 4a zusammen. Weiterhin vorteilhaft passen Ausführungsvariante A2 der Außenhülse 2 und die Innenhülse 4b zusammen.

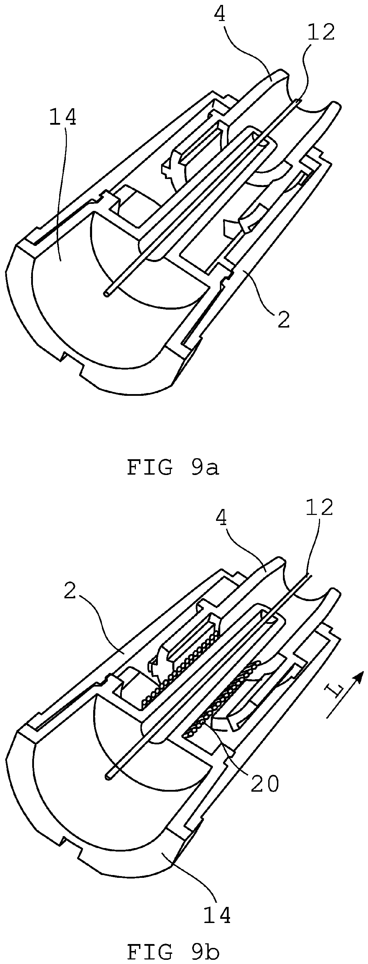

FIG 9a zeigt die Außenhülse 2 aus FIG 2 bzw. FIG 8 mit aufgenommener Innenhülse 4 aus FIG 5 in der Grundposition und das Kanülträgerelement 14 mit Kanüle 12 aus FIG 7 in einer Schnittansicht mit Schnittebene auf der Rotationsachse. Vom Betrachter aus gesehen vorn bzw. unten ist das Kanülträgerelement 14 mit Kanüle 12 angeordnet. Auf dem Kanülträgerelement 14 ist die Außenhülse 2 angeordnet und fixiert. Auf das Kanülträgerelement 14 wiederum ist die Innenhülse 4 aufgeschoben, die gleichzeitig zum Teil in die Außenhülse 2 aufgenommen ist und zum Teil aus der Außenhülse 2 nach oben hin herausragt. Die Kanüle 12 ist wenigstens aus der Außenhülse 2 und der Innenhülse 4 herausstehend angeordnet.

FIG 9b zeigt die FIG 9a mit zusätzlich aufgenommenem Rückstellelement 20 aus FIG 6, ebenfalls in der Schnittansicht. Das Rückstellelement 20 in Form einer Spiralfeder ist dabei zwischen Kanülträgerelement 14 und Innenhülse 4 angeordnet. Es erstreckt sich konzentrisch zum zylindrischen Bereich des Kanülträgerelement 14 und schlägt unten am Kanülträgerelement 14 und oben an der Innenhülse 4 an, sodass Kanülträgerelement 14 und Innenhülse 4 von dem Rückstellelement 20 beabstandet zueinander angeordnet sind. Das Rückstellelement 20 übt vorteilhaft als Druckfeder eine Rückstellkraft auf die Innenhülse 4 aus, die eine Linearverschiebung L innerhalb der Außenhülse 2 durchführbar macht.

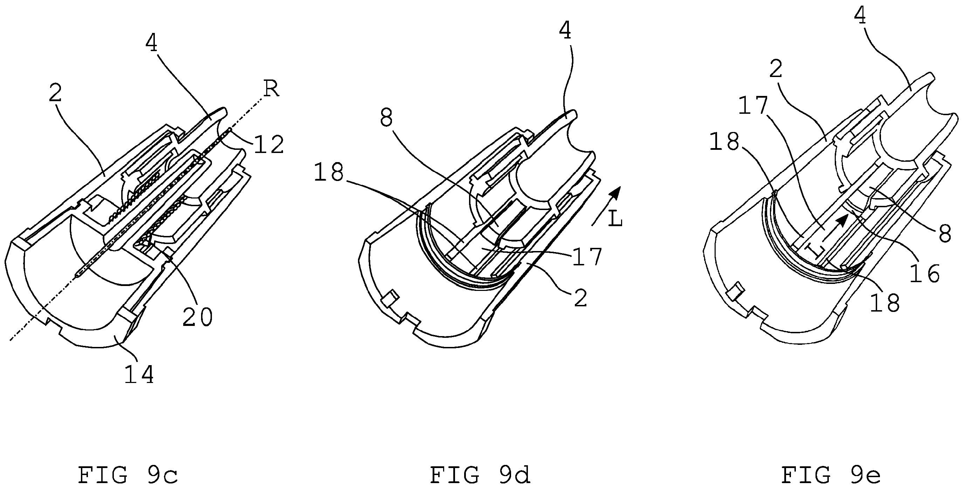

FIG 9c zeigt eine Schnittansicht der Applikationsposition kurz vor der Sicherungsposition. Die Innenhülse 4 ist um die Rotationsachse R rotiert und von einer ersten Führungsnut 17 über die Rampe R₂ (nicht gezeigt) in eine zweite Führungsnut 17 überführt.

FIG 9d zeigt die Schnittansicht aus FIG 9c reduziert auf Außen- und Innenhülse 2, 4. Diese Darstellung dient dem besseren Verständnis der mechanischen Interaktion zwischen Außen- und Innenhülse 2, 4. Das Federelement 8 der Innenhülse 4 greift zum Teil formschlüssig in die Führungsnut 17 zwischen zwei Führungsstegen 18 ein.

FIG 9e zeigt die gezeigte Schnittansicht aus FIG 9d in der Sicherungsposition. Hierbei ist das Federelement 8 entlang der Linearverschiebung L in der Führungsnut 17 über den zweiten Arretierungsvorsprung 16 geschoben, sodass dieser das Federelement 8 untergreift und an einer Bewegung zurück hindert. Die Innenhülse 4 ragt dabei zum größten Teil vom Betrachter aus gesehen oben aus der Außenhülse 2 hinaus. Durch den Arretierungsvorsprung 16 verbleibt die Innenhülse 4 gegenüber der Außenhülse 2 in dieser Sicherungsposition.

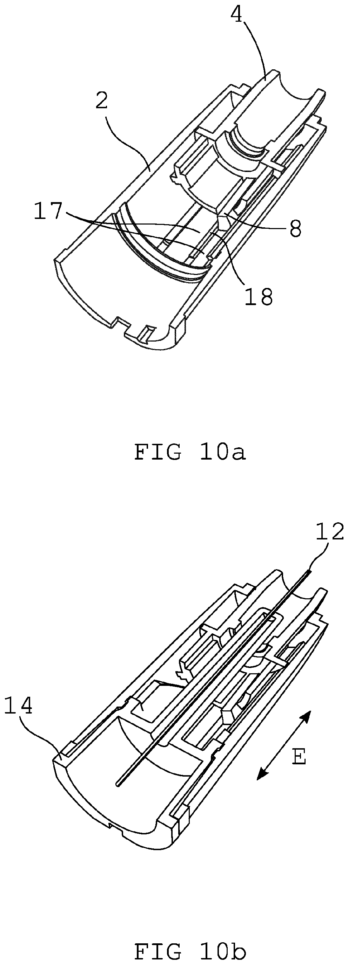

FIG 10a zeigt eine Schnittansicht einer alternativen Ausführungsform der Innenhülse 4b und der Außenhülse aus FIG 8b in der Grundposition. Hierbei greift das Federelement 8 in eine erste Führungsnut 17 ein.

FIG 10b zeigt eine Schnittansicht aus Fig. 10a, wobei hier das Kanülträgerelement 14 mit daran angeordneter Kanüle 12 zusätzlich gezeigt ist.

Die Kanüle 12 erstreckt sich entlang der Längserstreckung E der Außenhülse 2 bis nach oben, passiert dabei die in der Außenhülse 2 zum Teil aufgenommene Innenhülse 4 und ragt über diese nach oben hin hinaus. In dieser Grundposition ragt insgesamt betrachtet die Kanüle 12 sowohl über die Außen- als auch die Innenhülse 2, 4 hinaus, sodass mit der Kanüle 12 bspw. in die Haut eines Patienten eingestochen werden kann.

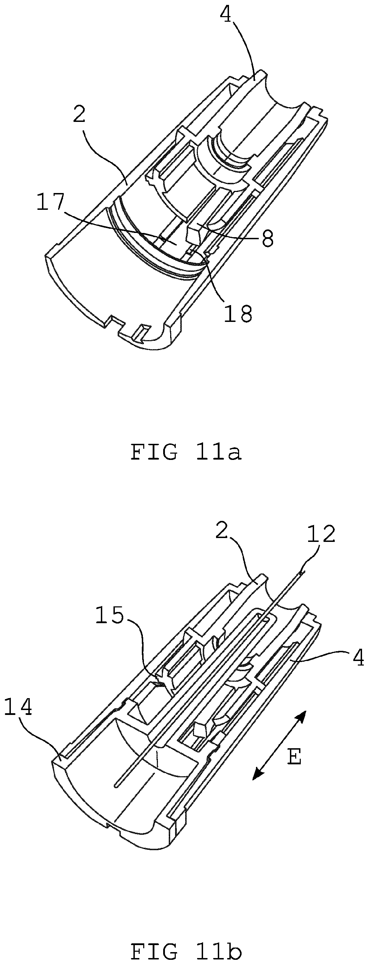

FIG 11a zeigt eine Schnittansicht aus Fig. 10a nunmehr den Übergang in die Applikationsposition. Hierbei gleitet das Federelement 8 der Innenhülse 4 auf und dann über die Rampe R₂ des einen Führungsstegs 18. Eine Rotation der Innenhülse 4 wird durchgeführt. Das Federelement 8 wird radial nach innen ausgelenkt, während es über den Führungssteg 18 geführt wird.

FIG 11b zeigt wiederrum Fig. 11a mit zusätzlichem Kanülträgerelement 14. Es ist hierbei deutlich zu erkennen, dass der Vorsprung 15 der Innenhülse 4 entlang der Führungsfläche an dem Trägerelement 14 entlang gleitet und die Innenhülse 4 entsprechend um ihre Achse rotiert. Die Innenhülse 4 rotiert dabei auch um die Kanüle 12 herum.

Die Applikationsposition ist erreicht, wenn das Federelement 8 der Innenhülse 4 nicht mehr auf dem Führungssteg 18, sondern in der zweiten Führungsnut 17 gleitet. Die Innenhülse 4 schnappt mit dem Federelement 8 in die Applikationsposition ein. Da das Federelement 8 hierbei zum Teil formschlüssig in der Führungsnut 17 verbracht ist, kann die Innenhülse 4 nicht mehr rotiert, sondern nur noch linear entlang der Längserstreckung der Außenhülse 2 verschoben werden. Zugleich wird das Rückstellelement (nicht gezeigt) komprimiert oder weiter komprimiert.

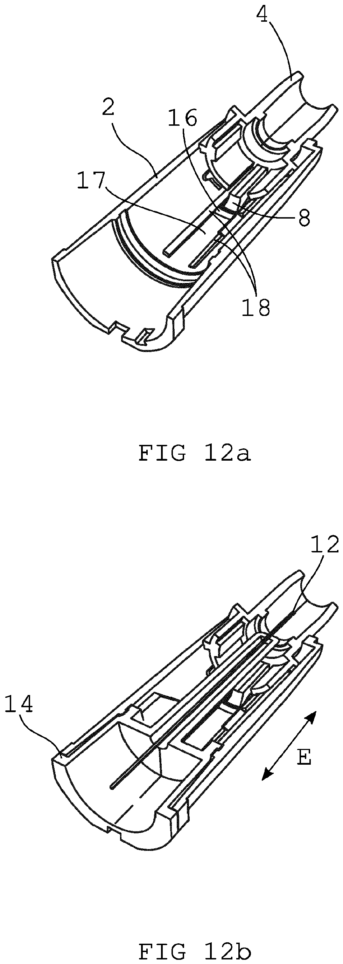

FIG 12a zeigt eine Schnittansicht einer alternativen Ausführungsform der Innen- und Außenhülse 4, 2 in der Sicherungsposition. Hierbei untergreift der Arretierungsvorsprung 16, welcher quer in der Führungsnut 17 angeordnet ist, das Federelement 8 der Innenhülse 4 derart, dass die Innenhülse 4 nicht mehr entlang der Führungsnut 17 zurückgeschoben werden kann. Gleichzeitig wird durch den teilweisen Formschluss des Federelements 8 in der Führungsnut 17 bzw. zwischen den Führungsstegen 18 die Innenhülse 4 an einer Rotation gehindert. Die Innenhülse 4 befindet sich somit in einer Sicherungsposition, in der sie verbleibt und durch äußere Kraftbeaufschlagung nicht mehr wegbewegt werden kann. Die Innenhülse 4 ragt dabei maximal aus der Außenhülse 2 vom Betrachter aus gesehen nach oben hinaus. Die axiale Verschiebung über den Arretierungsvorsprung 16 wird durch die Rückstellkraft des Rückstellelement unter zeitgleicher Beendigung der Kraftbeaufschlagung auf der Rückstellelement durchgeführt. Die Rückstellkraft reicht aus, damit das wenigstens eine Federelement 8 über den Arretierungsvorsprung 16 geführt und dann von diesem untergriffen werden.

FIG 12b zeigt eine Schnittansicht einer alternativen Ausführungsform der Innen- und Außenhülse 4, 2 mit daran angeordnetem Kanülträgerelement 14 mit Kanüle 12 in der Sicherungsposition. Diese Abbildung entspricht der FIG 12a mit Kanülträgerelement 14 und Kanüle 12. Es ist deutlich zu sehen, dass die Innenhülse 4 in der Sicherungsposition die Kanüle 12 an ihrem oberen Ende überragt, sodass die obere Spitzer der Kanüle 12 sicher in der Innenhülse 4 aufgenommen ist. Ein versehentliches Stechen ist nicht mehr möglich.

Obwohl die Erfindung im Detail durch die vorteilhaften Ausführungsbeispiele näher illustriert und beschrieben wurde, so ist die Erfindung nicht durch die offenbarten Beispiele eingeschränkt. Andere Variationen können vom Fachmann hieraus abgeleitet werden, ohne den Schutzumfang der Erfindung zu verlassen. Insbesondere beschränkt sich die Erfindung nicht auf die nachfolgend angegebenen Merkmalskombinationen, sondern es können auch für den Fachmann offensichtlich ausführbare andere Kombinationen und Teilkombinationen aus den offenbarten Merkmalen gebildet werden.

### Bezugszeichenliste

- 1: Sicherheitskanüleneinheit/Einheit
- 2: Außenhülse
- 2a: Verpackung
- 2b: Verdickung
- 2c: distale Öffnung
- 4: Innenhülse
- 5: Vorsprung
- 6: Vorsprung
- 7: Öffnung Innenhülse
- 8: Federelement
- 10: Führungsnase
- 12: Kanüle
- 13: Vorsprung
- 14: Kanülträgerelement
- 15: Vorsprung
- 15a: Materialverdickung
- 16: Arretierungsvorsprung
- 17: Führungsnut
- 18: Führungssteg
- 19: Basis
- 20: Rückstellelement
- R: Rotationsachse
- L: Linearverschiebung
- Z: zylindrischer Bereich
- B: Bereich
- E: Längserstreckung
- R₂: Rampe
- M: Mantelfläche
- A1, A2: Ausführungsvariante
- F1 - F6: Führungsflächen

## Patentansprüche

1. Sicherheitskanüleneinheit (1) zur verletzungsfreien Entsorgung gebrauchter Injektionskanülen wenigstens aufweisend
a.ein Kanülträgerelement (14), welches wenigstens eine Kanüle (12) aufweist,
b. eine Außenhülse (2) zur Aufnahme des Kanülträgerelements (14), wobei Kanülträgerelement (14) und Außenhülse (2) miteinander gekoppelt sind und die Außenhülse (2) weiterhin eine distale Öffnung (2c) aufweist,
c. sowie wenigstens eine Innenhülse (4), welche gegenüber der Außenhülse (2) axial verschiebbar (L) und zumindest teilweise um eine Rotationsachse (R) rotierbar ausgebildet ist, und welche zumindest nach Gebrauch der Sicherheitskanüleneinheit (1) mittels wenigstens eines Rückstellelements (20) abschnittsweise durch die distale Öffnung (2c) der Außenhülse (2) herausragend angeordnet und die gebrauchte Sicherheitskanüle (12) überspannt, wobei die Innenhülse (4) über wenigstens einen Sicherungsmechanismus in dieser Endposition gehalten ist.

2. Sicherheitskanüleneinheit nach Anspruch 1,
**dadurch gekennzeichnet, dass**
die Innenhülse (4) zwischen Außenhülse (2) und Kanülträgerelement (14) angeordnet ist.

3. Sicherheitskanüleneinheit nach wenigstens einem der vorangegangenen Ansprüche
**dadurch gekennzeichnet, dass**
das Kanülträgerelement (14) wenigstens einen in Längsrichtung (E) der Sicherheitskanüleneinheit (1) gerichteten Vorsprung (13) mit einer in Umfangsrichtung abgeschrägten Führungsfläche (F) aufweist, wobei Führungsfläche (F) und ein freies Ende der Innenhülse (4) miteinander in Wirkverbindung stehen.

4. Sicherheitskanüleneinheit nach Anspruch 3,
**dadurch gekennzeichnet, dass**
die Innenhülse (4) bei Kraftbeaufschlagung entlang der wenigstens einen abgeschrägten Führungsfläche (F) bewegbar ist und sich während der Bewegung entlang der Führungsfläche (F) wenigstens eine Drehbewegung der Innenhülse (4) um die Rotationsachse (R) und/oder eine axiale Verschiebung der Innenhülse (4) gegenüber der Außenhülse (2) ausbildet.

5. Sicherheitskanüleneinheit nach Anspruch 1,
**dadurch gekennzeichnet, dass**
die Außenhülse (2) an einer inneren Mantelfläche (M), welche der Innenhülse (4) zugewandt ist, wenigstens in Längsrichtung (E) der Außenhülse verlaufende Führungsstege (18) und/oder Vorsprünge aufweist, wobei zwischen zueinander benachbart angeordneten Führungsstegen (18) Führungsnuten (17) ausgebildet sind, und die Innenhülse (4) an einer äußeren Mantelfläche, welche der inneren Mantelfläche (M) der Außenhülse (2) zugewandt ist, wenigstens ein radial nach außen gerichtetes Federelement (8) aufweist.

6. Sicherheitskanüleneinheit nach wenigstens einem der vorgenannten Ansprüche,
**dadurch gekennzeichnet, dass**
die Innenhülse (4) wenigstens zwei einander diametral gegenüberliegende Federelemente (8) aufweist, welche bei axialer Verschiebung der Innenhülse (4) in den Führungsnuten (17) der Außenhülse (4) und bei Rotationsbewegung der Innenhülse (4) über Führungsstege (18, R₂) der Außenhülse (2) hinweg rotierbar sind.

7. Sicherheitskanüleneinheit nach Anspruch 4,
**dadurch gekennzeichnet, dass**
der Sicherungsmechanismus nach Gebrauch der Kanüle (12) durch eine formschlüssige und/oder kraftschlüssige Verbindung zwischen Federelement (8) und wenigstens einem Arretierungsvorsprung (R₂; 16) ausgebildet ist, wobei der wenigstens eine Arretierungsvorsprung (R₂; 16) an der inneren Mantelfläche (M) der Außenhülse (2) angeordnet ist.

8. Sicherheitskanüleneinheit nach Anspruch 4,
**dadurch gekennzeichnet, dass**
das Rückstellelement (20) in einer Ausgangsposition bereits vorgespannt ausgebildet ist.

9. Einhandverfahren zur Betätigung eines Wirkstoffverabreichungsmittels, welches wenigstens eine Sicherheitskanüleneinheit (1) nach wenigstens einem der vorgenannten Ansprüche, wobei die Sicherheitskanüleneinheit (1) an dem Wirkstoffverabreichungsmittel distal angeordnet ist und die Innenhülse (4) mit dem zumindest teilweise darin aufgenommenen Kanülträgerelement (14) durch eine Druckkraftbeaufschlagung auf die Innenhülse (4) von einer Grundposition in eine Applikationsposition und durch anschließendes Beenden der Druckkraftbeaufschlagung weiter in eine Sicherungsposition verbracht wird, von der die Innenhülse (4) nicht mehr zurückbewegt werden kann.

10. Wirkstoffverabreichungsmittel mit einer Sicherheitskanüleneinheit (1) nach wenigstens einem der vorangegangen Ansprüche.

11. Verwendung der Sicherheitskanüleneinheit (1) nach wenigstens einem der Ansprüche 1 bis 9 zur Bestückung von Insulinspritzen, Insulinpens oder dergleichen.
